(19) Europäisches Patentamt
European Patent Office
Office européen des brevets

(11) Veröffentlichungsnummer: **0 173 918**
**A2**

(12) # EUROPÄISCHE PATENTANMELDUNG

(21) Anmeldenummer: **85110547.8**

(22) Anmeldetag: **22.08.85**

(51) Int. Cl.⁴: **C 07 D 233/56**
C 07 D 249/08, A 01 N 43/50
A 01 N 43/653

(30) Priorität: **03.09.84 JP 182579/84**

(43) Veröffentlichungstag der Anmeldung:
**12.03.86 Patentblatt 86/11**

(84) Benannte Vertragsstaaten:
**AT BE CH DE FR GB IT LI NL SE**

(71) Anmelder: **NIHON TOKUSHU NOYAKU SEIZO K.K.**
**No.4, 2-chome, Nihonbashi Honcho Chuo-ku**
**Tokyo 103(JP)**

(72) Erfinder: **Saito, Junichi**
**3-7-12, Osawa**
**Nitaka-shi Tokyo(JP)**

(72) Erfinder: **Tamura, Tatsuo**
**1-7-30, Hanenaka Hamura-machi**
**Nishitama-gun Tokyo(JP)**

(72) Erfinder: **Kurahashi, Yoshio**
**47-15, Oya-machi**
**Hachioji-shi Toyko(JP)**

(72) Erfinder: **Uzawa, Shigeru**
**3026-1, Kamitsuruma**
**Sagamihara-shi Kanagawa-ken(JP)**

(72) Erfinder: **Matsumoto, Noboru**
**39-15, Namiki-cho**
**Hachioji-shi Tokyo(JP)**

(72) Erfinder: **Yamaguchi, Naoko**
**4-6-7, Shinmei**
**Hino-shi Tokyo(JP)**

(74) Vertreter: **Schumacher, Günter, Dr. et al,**
**c/o Bayer AG Konzernverwaltung RP Patentabteilung**
**D-5090 Leverkusen 1 Bayerwerk(DE)**

(54) **Substituierte Phenylsulfonylazole.**

(57) Die Erfindung betrifft neue substituierte Phenylsulfonylazole, ein Verfahren zu ihrer Herstellung und ihre Verwendung als Fungizide in Landwirtschaft und Gartenbau.

Die Verbindungen der Formal (I)

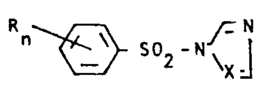

(I)

in der X, R und n die in der Beschreibung angegebene Bedeutung haben, wurden durch Reaktion eines substituierten Benzolsulfonylhalogenids mit einem Azol hergestellt.

Die Verbindungen der Formel (I) zeigen eine hohe Aktivitat gegen phytopathogene Fungi.

EP 0 173 918 A2

Croydon Printing Company Ltd

## Substituierte Phenylsulfonylazole

Die vorliegende Erfindung betrifft neue substituierte Phenylsulfonylazole, ein Verfahren zu ihrer Herstellung und ihre Verwendung als Fungizide in Landwirtschaft und Gartenbau.

Es wurde bereits offenbart, daß bestimmte Phenylsulfonylimidazol-Derivate als Bleichmittel geeignet sind (JP-OS Nr. 64181/1979 bzw. US-PS 4 115 060). Weiterhin besitzt gemäß dem Stand der Technik 2-Trichloromethyl-4-nitrophenyl-sulfonylimidazol fungizide Aktivität (vgl. EP-A 0 044 394).

Numehr wurden neue substituierte Phenylsulfonylazole der Formel (I) gefunden

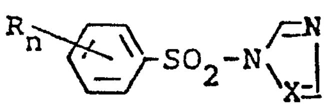

(I)

,

in der

X N oder eine CH-Gruppe bezeichnet,

R Halogen, Niederalkyl, Niederalkoxy, Trifluoromethyl, Nitro, gegebenenfalls mit Halogen substituiertes Phenyl oder Cycloalkyl bezeichnet und

n 2 oder 3 bezeichnet und weiterhin in dem Fall, in dem R nicht eine Methyl-Gruppe ist, auch 1 bezeichnet.

Substituierte Phenylsulfonylazole der Formel (I) werden erhalten, wenn substituierte Benzolsulfonylhalogenide der Formel (II)

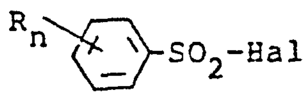

(II)

,

in der R und n die im Vorstehenden angegebenen Bedeutungen haben und Hal ein Halogen-Atom bezeichnet, mit Azolen der allgemeinen Formel (III)

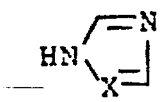

(III)

,

in der X die angegebene Bedeutung hat, umgesetzt werden, gegebenenfalls in Gegenwart von Säure-Acceptoren und inerten Lösungsmitteln.

Die neuen substituierten Phenylsulfonylazole zeigen potente land- und gartenwirtschaftliche fungizide Eigenschaften.

Überraschenderweise zeigen die substituierten Phenylsulfonylazole gemäß der vorliegenden Erfindung eine wesentlich stärkere fungizide Wirkung in Landwirtschaft und Gartenbau als die aus dem Stand der Technik bekannten und sind nicht phytotoxisch.

Unter den substituierten Phenylsulfonylazolen der Formel (I) sind bevorzugte Verbindungen diejenigen, in denen

X eine CH-Gruppe oder Stickstoff bezeichnet,

R Chlor, Brom, Alkyl mit 1 bis 4 Kohlenstoff-Atomen, Alkoxy mit 1 bis 4 Kohlenstoff-Atomen, Nitro, Phenyl und/oder Cycloalkyl mit 5 bis 6 Kohlenstoff-Atomen bezeichnet und

n 2 oder 3 bezeichnet und weiterhin in dem Fall, in dem R nicht eine Methyl-Gruppe ist, auch 1 bezeichnet.

Besonders bevorzugte substituierte Phenylsulfonylazole der Formel (I) sind diejenigen, in denen

X eine CH-Gruppe bezeichnet,

R Chlor, Brom, Methyl, Ethyl, Nitro oder Phenyl bezeichnet und

n 2 bezeichnet und weiterhin in dem Fall, in dem R nicht eine Methyl-Gruppe ist, auch 1 bezeichnet.

Ganz besonders bevorzugt sind solche Verbindungen gemäß der vorliegenden Erfindung, in denen

X eine CH-Gruppe bezeichnet,

R Methyl, Nitro, Brom und Chlor bezeichnet und

n den Zahlenwert 2 bezeichnet und

die beiden Substituenten R sich in den 2- und 5-Stellungen befinden.

Speziell erwähnt seien die folgenden Verbindungen:

1-(5-Nitro-2-methyl-phenylsulfonyl)imidazol,

1-(2,5-Dimethyl-phenylsulfonyl)imidazol,

1-(2-Biphenylylsulfonyl)imidazol,

1-(5-Bromo-2-methyl-phenylsulfonyl)imidazol.

Die Herstellung einer Verbindung der allgemeinen Formel (I) läßt sich wie folgt erläutern: Wenn beispielsweise 5-Nitro-2-methyl-phenylsulfonylchlorid und Imidazol als Ausgangsstoffe eingesetzt werden, kann der Reaktionsablauf durch die folgende Reaktionsgleichung dargestellt werden:

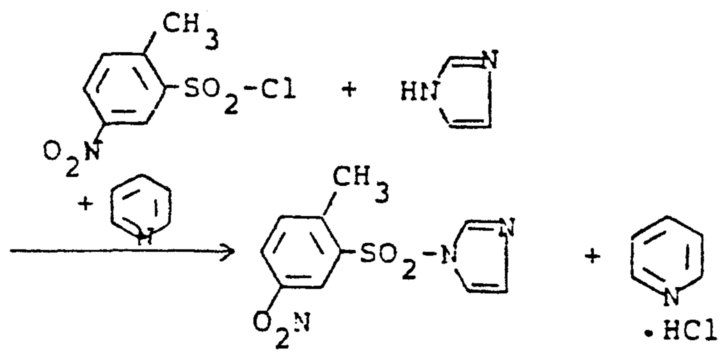

Die Formel (II) gibt eine allgemeine Definition der substituierten Benzolsulfonylhalogenide, die als Ausgangsstoffe für die Synthese der Verbindungen gemäß der vorliegenden Erfindung benötigt werden. In dieser Formel haben R und n vorzugsweise die bereits im Vorstehenden angegebenen Bedeutungen. Hal bezeichnet vorzugsweise Chlor.

Die Verbindungen der Formel (II) sind bereits wohlbekannt (wegen Einzelheiten vgl. beispielsweise Beilstein's Handbuch der organischen Chemie (Hauptwerk) 11, ab S. 34, sowie die entsprechenden Kapitel in den Ergänzungswerken).

Als Beispiele seien erwähnt:
2,5-Dichlorobenzolsulfonylchlorid,
2-Isopropylbenzolsulfonylchlorid,
2,5-Xylolsulfonylchlorid,

5-Bromo-2-toluolsulfonylchlorid,
5-Chloro-2-toluolsulfonylchlorid,
2-Phenylbenzolsulfonylchlorid,
5-Chloro-2-methoxybenzolsulfonylchlorid,
5-Nitro-2-toluolsulfonylchlorid und
5-Chloro-2-cyclohexylbenzolsulfonylchlorid.

Die Azol-Verbindungen der allgemeinen Formel (III), die in gleicher Weise Ausgangsstoffe sind, sind Imidazol und 1,2,4-Triazol. Die Verbindungen sind wohlbekannt.

Das Verfahren zur Herstellung der Verbindungen der Formel (I) gemäß der vorliegenden Erfindung kann zweckmäßigerweise in einem Lösungsmittel oder Verdünnungsmittel durchgeführt werden. Zu diesem Zweck können inerte organische Lösungs- und Verdünnungsmittel verwendet werden. Beispiele für solche Lösungsmittel oder Verdünnungsmittel umfassen:

Aliphatische, alicyclische und aromatische Kohlenwasserstoffe, die gegebenenfalls chloriert sein können, wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol, Ether wie Diethylether, Methylethylether, Di-isopropylether, Dibutylether, Propylenoxid, Dioxan und Tetrahydrofuran, Ketone wie Aceton, Methylethylketon, Methylisopropylketon und Methylisobutylketon, Nitrile wie Acetonitril, Propionitril und Acrylnitril, Alkohole wie Methanol, Ethanol, Isopropanol, Butanol und Ethylenglycol, Ester wie Ethylacetat und Amylacetat, Säureamide wie Dimethylformamid und Dimethylacetamid, Sulfone und Sulfoxide wie Dimethylsulfoxid und Sulfolan sowie Basen wie Pyridin.

Die Reaktion gemäß der Erfindung kann in Gegenwart eines Säure-Acceptors durchgeführt werden. Beispiele für die Säure-Acceptoren sind die Hydroxide, Carbonate, Hydrogencarbonate und Alkoholate von Alkalimetallen sowie tertiäre Amine wie Triethylamin, Diethylanilin und vorzugsweise Pyridin.

Das obige Verfahren kann in einem weiten Temperaturbereich durchgeführt werden. Im allgemeinen kann es bei einer Temperatur zwischen etwa -20°C und dem Siedepunkt der Mischung, vorzugsweise zwischen etwa 0°C und etwa 100°C, durchgeführt werden. Die Reaktion wird zweckmäßigerweise unter Umgebungsdruck durchgeführt, jedoch ist es auch möglich, unter erhöhtem oder vermindertem Druck zu arbeiten.

In dieser Reaktion werden etwa 1 bis 3 mol der Azol-Verbindung der Formel (III) auf 1 mol des substituierten Benzolsulfonylhalogenids der Formel (II) eingesetzt. Die Aufarbeitung der Reaktionsprodukte kann in üblicher und allgemein bekannter Weise erfolgen.

Die aktiven Substanzen gemäß der vorliegenden Erfindung zeigen leistungsfähige fungizide Effekte auf den Gebieten der Landwirtschaft und des Gartenbaus. Aus diesem Grunde können sie als Fungizide in Landwirtschaft und Gartenbau eingesetzt werden.

Fungizide Mittel werden im Pflanzenschutz eingesetzt zur Bekämpfung von Plasmodiophoromycetes, Oomycetes, Chytridiomycetes, Zygomycetes, Ascomycetes, Basidiomycetes und Deuteromycetes.

Bakterielle Mittel werden im Pflanzenschutz eingesetzt zur Bekämpfung von Pseudomonadaceae, Rhizobiaceae, Enterobacteriaceae, Corynebacteriaceae und Streptomycetaceae.

Einige der fungale und bakterielle Erkrankungen verursachenden Organismen, die unter die oben aufgeführten generischen Namen fallen, seien hier als Beispiel aufgeführt, die jedoch nicht als Beschränkung zu verstehen sind:
Botrytis-Species wie beispielsweise Botrytis cinerea;
Plasmopara-Species wie beispielsweise Plasmopara viticola;
Uromyces-Species wie beispielsweise Uromyces appendiculatus;
Sphaerotheca-Species wie beispielsweise Sphaerotheca fuliginea;
Venturia-Species wie beispielsweise Venturia inaequalis;
Podosphaera-Species wie beispielsweise Podosphaera leucotricha,
Phytophthora-Species wie beispielsweise Phytophthora infestans;
Erysiphe-Species wie beispielsweise Erysiphe graminis;
Puccinia-Species wie beispielsweise Puccinia recondita;
Fusarium-Species wie beispielsweise Fusarium culmorum;
Ustilago-Species wie beispielsweise Ustilago nuda oder Ustilago avenae;
Septoria-Species wie beispielsweise Septoria nodorum;
Tilletia-Species wie beispielsweise Tilletia caries;
Xanthomonas-Species wie beispielsweise Xanthomonas oryzae;
Pseudomonas-Species wie beispielsweise Pseudomonas lachrymans;

Piricularia-Species wie beispielsweise Piricularia oryzae;
Pellicularia-Species wie beispielsweise Pellicularia sasakii;
Pyrenophora-Species wie beispielsweise Pyrenophora teres (Conidia Form Drechslera; Syn. Helminthosporium);
Cochliobolus-Species wie beispielsweise Cochliobolus sativus (Conidia Form Drechslera; Syn. Helminthosporium);
Cercospora-Species wie beispielsweise Cercospora canescens.

Insbesondere zeigen die neuen Verbindungen eine sehr hervorragende Bekämpfungswirksamkeit gegen die Braunfäule der Tomate (Phytophthora infestans). Weiterhin zeigen sie gute Aktivität gegen die Brusone-Krankheit von Reis (Piricularia oryzae), falschen Mehltau der Gurken und Melonen (Pseudoperonospora cubensis) sowie falschen Mehltau des Weinstocks (Reben-Peronospora; Plasmopara viticola).

Als Fungizide für Landwirtschaft und Gartenbau können die Verbindungen der Formel (I) gemäß der vorliegenden Erfindung unmittelbar nach Verdünnen mit Wasser oder aber in Form verschiedener Formulierungen eingesetzt werden, wie sie unter Verwendung landwirtschaftlich unbedenklicher Hilfsstoffe durch Verfahren gewonnen werden, die bei der praktischen Herstellung von Agrochemikalien allgemein angewandt werden. Im praktischen Einsatz werden diese Präparate entweder unmittelbar oder nach dem Verdünnen mit Wasser auf die gewünschte Konzentration zur Anwendung gebracht.

Die landwirtschaftlich unbedenklichen Hilfsstoffe, auf die hier Bezug genommen wird, umfassen beispielsweise Verdünnungsmittel (Lösungsmittel, Streckmittel, Träger), oberflächenaktive Mittel (Lösungsvermittler, Emulgatoren, Dispergiermittel, Netzmittel), Stabilisatoren, Haftmittel, Aerosol-Treibmittel und synergistische Mittel.

Beispiele für die Lösungsmittel sind Wasser, weiterhin organische Lösungsmittel wie Kohlenwasserstoffe, beispielsweise n-Hexan, Petrolether, Erdöl-Fraktionen, z.B. Paraffinwachse, Kerosin, Leichtöle, Mittelöle und Schweröle, Benzol, Toluol und Xylol, weiterhin halogenierte Kohlenwasserstoffe wie Methylenchlorid, Kohlenstofftetrachlorid, Ethylenchlorid, Ethylendibromid, Chlorbenzol und Chloroform, Alkohole wie Methanol, Ethanol, Propanol und Ethylenglycol, Ether wie Diethylether, Ethylenoxid und Dioxan, Alkohol-ether wie Ethylenglycol-monomethylether, Ketone wie Aceton und Isophoron, Ester wie Ethylacetat und Amylacetat, Amide wie Dimethylformamid und Dimethylacetamid sowie Sulfoxide wie Dimethylsulfoxid.

Beispiele für die Streckmittel oder Träger umfassen anorganische Pulver, beispielsweise Schwefel, Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (wie Pyrophyllit, Talkum, Montmorillonit, Beidellit, Vermikulit, Kaolinit und Glimmer), pflanzliche Pulver wie Getreidepulver, Stärkearten, verarbeitete Stärkearten, Zucker, Glucose und zerkleinerte Stengel von Pflanzen, sowie Pulver aus synthetischen

Harzen wie Phenol-Harzen, Harnstoff-Harzen und Vinylchlorid-Harzen.

Beispiele für oberflächenaktive Mittel umfassen anionische oberflächenaktive Mittel wie Alkylsulfonsäureester (wie Natriumlaurylsulfat), Arylsulfonsäuren (wie Alkylarylsulfonsäure-Salze und Natriumalkylnaphthalinsulfonate), Bernsteinsäure-Salze und Salze von Schwefelsäureestern von Polyethylenglycol-alkylarylethern, kationische oberflächenaktive Mittel wie Alkylamine (z.B. Laurylamin, Stearyltrimethylammoniumchlorid und Alkyldimethylbenzylammoniumchlorid) und Polyoxyethylenalkylamine, nicht-ionische oberflächenaktive Mittel wie Polyoxyethylenglycolether (z.B. Polyoxyethylenalkylarylether und deren Kondensationsprodukte), Polyoxyethylenglycolester (z.B. Polyoxyethylenfettsäureester) und Ester mehrwertiger Alkohole (z.B. Polyoxyethylensorbitan-monolaurat) sowie amphotere oberflächenaktive Mittel.

Beispiele für andere Hilfsstoffe umfassen Stabilisatoren, Haftmittel (z.B. landwirtschaftliche Seifen, Casein-Kalk, Natriumalginat, Polyvinylalkohol, Haftmittel vom Vinylacetat-Typ und Acryl-Haftmittel), Aerosol-Treibmittel (wie Trichlorofluoromethan, Dichlorofluoromethan, 1,2,2-Trichloro-1,1,2-trifluoroethan, Chlorbenzol und niedere Ether), die Verbrennung steuernde Mittel für Räuchermittel (wie Nitrite, Zink-Pulver und Dicyandiamid), Sauerstoff abgegebende Mittel (wie Chlorate), wirkungsverlängernde Mittel, Dispersions-Stabilisatoren (wie Casein, Tragant, Carboxymethylcellulose und Polyvinylalkohol) und synergistische Mittel.

Die Verbindungen gemäß der vorliegenden Erfindung können mittels allgemein bei der Herstellung von Agrochemikalien angewandter Methoden zu verschiedenartigen Präparaten formuliert werden. Erläuternde Beispiele für solche Anwendungsformen sind emulgierbare Konzentrate, Öl-Präparate, benetzbare Pulver, lösliche Pulver, Suspensionen, Stäubemittel, Granulat, Pulverpräparate, Räuchermittel, Tabletten, Aerosole, Pasten und Kapseln.

Das Fungizid für Landwirtschaft und Gartenbau gemäß der vorliegenden Erfindung kann etwa 0,1 bis etwa 95 Gew.-%, vorzugsweise etwa 0,5 bis etwa 90 Gew.-% des vorerwähnten aktiven Wirkstoffes enthalten.

Für den praktischen Gebrauch beträgt die geeignete Menge der aktiven Verbindung in den vorgenannten Mitteln in ihren verschiedenen Formen und gebrauchsfertigen Präparaten im allgemeinen etwa 0,0001 bis etwa 20 Gew.-%, vorzugsweise etwa 0,005 bis etwa 10 Gew.-%.

Der Gehalt des Wirkstoffs kann in geeigneter Weise je nach der Art der Formulierung, dem Verfahren, Zweck, der Zeit und dem Ort seiner Anwendung, dem Zustand des Auftretens der Krankheit etc. variiert werden.

Erforderlichenfalls können die Verbindungen gemäß der vorliegenden Erfindung in Kombination mit anderen Agrochemikalien verwendet werden, beispielsweise mit Insektiziden, anderen Fungiziden, Mitiziden, Nematiziden, Anti-Virus-Mitteln, Herbiziden, Pflanzen-Wachstumsregulatoren und Lockstoffen /‾Organophosphat-Verbindungen, Carbamat-Verbindungen, Dithio(oder thiol)carbamat-Verbindungen, organischen Chlor-Verbindungen, Dinitro-Verbindungen, organischen Schwefel- oder metallorganischen Verbindungen, Antibiotika, substituierten Diphenylether-Verbindungen, Harnstoff-Verbindungen und Triazin-Verbindungen_7 und/oder Düngemitteln.

Die den im Vorstehenden bezeichneten Wirkstoff der Erfindung enthaltenden verschiedenartigen Mittel und gebrauchsfertigen Präparate können mittels verschiedener Verfahren zur Anwendung gebracht werden, wie sie allgemein für das Aufbringen von Agrochemikalien gebräuchlich sind, beispielsweise durch Sprühen (Flüssigkeitssprühen, Einnebeln, Zerstäuben, Stäuben, Streuen von Granulat, Wasser-Oberflächenbehandlung, Gießen etc.), Bodenbehandlung (Vermischen, Streuen, Bedampfen und Gießen etc.), Oberflächenbehandlung (wie Beschichten, Bändern, Aufstäuben und Bedecken) und Tauchen. Sie können auch mittels des sogenannten Ultra-Low-Volume-Sprühverfahrens aufgebracht werden. Nach diesem Verfahren kann der Wirkstoff in einer Menge von nahezu 100 % zur Anwendung gelangen.

Die Aufwandmenge pro Flächeneinheit läßt sich in geeigneter Weise wählen und beträgt beispielsweise etwa 0,03 bis etwa 10 kg/ha, vorzugsweise etwa 0,3 bis etwa 6 kg/ha.

Gemäß der vorliegenden Erfindung kann ein fungizides Mittel für Landwirtschaft und Gartenbau verfügbar gemacht werden, das eine Verbindung der allgemeinen Formel (I) als Wirkstoff und ein Verdünnungsmittel (ein Lösungsmittel und/oder ein Streckmittel und/oder einen Träger) und/oder ein oberflächenaktives Mittel und, sofern weiter erforderlich, einen Stabilisator, ein Haftmittel und ein synergistisches Mittel enthält.

Die vorliegende Erfindung stellt ebenfalls ein Verfahren zur Bekämpfung von Pflanzenkrankheiten zur Verfügung, bei dem auf ein Pathogen und/oder den Ort seines Auftretens und/oder den Ort des Auftretens einer Pflanzenkrankheit eine Verbindung der allgemeinen Formel (I) entweder allein oder im Gemisch mit einem Verdünnungsmittel (einem Lösungsmittel und/oder einem Streckmittel und/oder einem Träger) und/oder einem oberflächenaktiven Mittel und, sofern weiter erforderlich, einem Stabilisator, einem Haftmittel und einem synergistischen Mittel etc. aufgebracht wird.

Die folgenden Beispiele erläutern die vorliegende Erfindung im einzelnen. Es sei jedoch darauf hingewiesen, daß die vorliegende Erfindung nicht allein auf diese speziellen Beispiele beschränkt ist.

Herstellungsbeispiele:

Beispiel 1

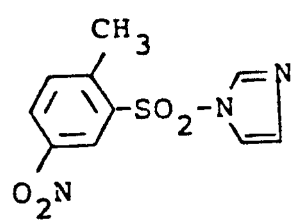

(Verbindung Nr. 1)

2,36 g 5-Nitro-2-methyl-phenylsulfonylchlorid (0,01 mol) und 1,36 g Imidazol (0,02 mol) wurden in 15 ml Pyridin gelöst, und die Mischung wurde zur Reaktion 3 h bei 50°C gerührt. Nach der Reaktion wurde die Reaktionsmischung in Wasser gegossen und mit Chloroform

extrahiert. Die Chloroform-Schicht wurde mit Wasser gewaschen und entwässert. Das Abdampfen des Chloroforms lieferte 1,70 g 1-(5-Nitro-2-methyl-phenylsulfonyl)-imidazol mit dem Schmp. 119-120°C.

Herstellung des Ausgangsmaterials

50 ml Chlorsulfonsäure wurden innerhalb von 30 min tropfenweise zu einer Lösung von 13,7 g p-Nitrotoluol in 50 ml Chloroform unter Eiskühlung hinzugefügt. Nach der Zugabe wurde die Mischung 6 h zum Rückfluß erhitzt. Nach dem Kochen unter Rückfluß wurde die Reaktionsmischung in Eiswasser gegossen und mit Chloroform extrahiert. Die Chloroform-Schicht wurde mit Wasser gewaschen und entwässert. Das Abdampfen des Chloroforms lieferte 15,5 g 5-Nitro-2-methyl-phenylsulfonylchlorid mit dem Schmp. 43-44°C.

Die folgenden Verbindungen der allgemeinen Formel

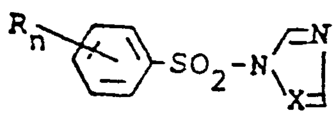

(I)

wurden nach der gleichen Methode, wie sie in Beispiel 1 beschrieben ist, erhalten.

| Verbindung Nr. | $R_n$ | x | Physikal. Konst. (Schmp., °C) |
|---|---|---|---|
| 2 | 2-Cl | Cl | 68 - 69 |
| 3 | 3-Cl | CH | 112 - 114 |
| 4 | $2,3-Cl_2$ | CH | 140 - 151 |
| 5 | $2,5-Cl_2$ | CH | 99 - 100 |
| 6 | $2,4,5-Cl_3$ | CH | 114 - 117 |
| 7 | $2-C_3H_7$-iso | CH | 72 - 77 |
| 8 | $2,4-(CH_3)_2$ | CH | 83 - 85 |
| 9 | $2,5-(CH_3)_2$ | CH | 123 - 124 |
| 10 | $2,4,6-(CH_3)_3$ | CH | 99 - 101 |
| 11 | $2-CH_3$, 5-Br | CH | 112 - 116 |
| 12 | $2-CH_3$, 5-Cl | CH | 88 -1 90 |
| 13 | $2-CH_3$, 4-F | CH | 84 - 87 |
| 14 | 2- 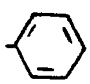 | CH | 111 - 113 |
| 15 | 4-Cl | N | 130 - 132 |
| 16 | 4-Br | N | 128 - 130 |
| 17 | $2,5-Cl_2$ | N | 139 - 151 |
| 18 | $2,4,5-Cl_3$ | N | 151 - 152 |
| 19 | $2-OCH_3$, 5-Cl | N | 147 - 149 |
| 20 | 2- 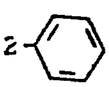 | N | 90 - 92 |
| 21 | 2- 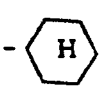 , 5-Cl | CH | Öl |
| 22 | $2-C_2H_5$, 5-Br | CH | 102 - 103 |

Verwendungsbeispiele:

Die bekannten Vergleichs-Verbindungen werden wie folgt identifiziert:

(A) 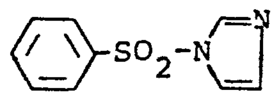

(B) 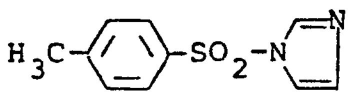

Diese Verbindungen sind in der JP-OS Nr. 64181/1979 bzw. der US-PS 4 115 060 beschrieben.

Beispiel A

Braunfäule der Tomate (Phytophthora infestans)

Eine Test-Verbindung in Form einer Emulsion, die gemäß dem nachfolgend beschriebenen Formulierungsbeispiel (b) hergestellt wurde, wurde mit Hilfe einer Spritzpistole auf Tomaten (Varietät: Kurihara) aufgesprüht, die in unglasierten 9 cm-Töpfen gezogen worden waren. Einen Tag nach dem Sprühen wurde mit einer Sporen-Suspension des oben genannten Pilzes inokuliert, und die Töpfe wurden über Nacht in einem Raum mit einer konstanten Temperatur von 22°C und einer Luftfeuchtigkeit von mehr als 90 % stehen gelassen. Fünf Tage später wurde der Grad der Erkrankung wie folgt aufgrund des prozentualen Anteils der Läsionen zeigenden Flächen bewertet, und der Bekämpfungsindex (%) wurde berechnet.

| Grad der Erkrankung | Verhältnis der Fläche der Verletzungen (%) |
|---|---|
| 0 | 0 |
| 0,5 | 2 oder weniger |
| 1 | 3 bis 5 |
| 2 | 6 bis 15 |
| 3 | 16 bis 30 |
| 4 | 31 bis 50 |
| 5 | 51 oder mehr |

$$\text{Bekämpfungs-Index (\%)} = \frac{\text{Grad der Erkrankung einer unbehandelten Fläche} - \text{Grad der Erkrankung einer behandelten Fläche}}{\text{Grad der Erkrankung einer unbehandelten Fläche}} \times 100$$

Als Ergebnis zeigten sämtliche der Verbindungen (1) bis (22) der vorliegenden Erfindung einen Bekämpfungs-Index von 100 %, wenn die Konzentration des Wirkstoffs 250 ppm betrug.

Zur Kontrolle wurden die Verbindungen (A) und (B) in der gleichen Weise wie oben angegeben getestet. Als Ergebnis zeigten bei einer Wirkstoff-Konzentration von 500 ppm die Verbindung (A) einen Bekämpfungs-Index von 40 % und die Verbindung (B) einen Bekämpfungs-Index von 50 %. Bei 250 ppm zeigten beide Vergleichs-Verbindungen einen Bekämpfungs-Index von 0 %.

Beispiel B

Brusone-Krankheit von Reis (Piricularia oryzae) / Behandlung des Laubs

Jede der Verbindungen (1) und (22) in Form eines benetzbaren Pulvers, das gemäß dem nachfolgend beschriebenen Formulierungsbeispiel (a) hergestellt wurde, wurde auf Reis-Pflanzen im 3- bis 4-Blatt-Stadium aufgesprüht. Am nächsten Tage wurde eine Suspension künstlich kultivierter Sporen des oben bezeichneten Pilzes zur Inokulierung auf die Reispflanzen (zweimal) aufgesprüht. Sieben Tage nach der Inokulierung wurden die Bekämpfungs-Indices (%) der Test-Verbindungen entsprechend dem üblichen Test der Bekämpfung der Brusone-Krankheit von Reis bestimmt. Es wurde gefunden, daß die Verbindungen Nr. 1 und Nr. 22 bei einer Wirkstoff-Konzentration von 500 ppm einen Bekämpfungs-Index von 100 % zeigten.

Formulierungsbeispiele

(a) Benetzbares Pulver

15 Teile der Verbindung Nr. 1 der Erfindung, 80 Teile eines Gemisches (1:5) aus pulvriger Diatomeenerde und Tonpulver, 2 Teile Natriumalkylbenzolsulfonat und 3 Teile Natriumalkylnaphthalinsulfonat/Formaldehyd-Kondensat werden pulverisiert und zu einem benetzbaren Pulver vermischt. Dieses wird mit Wasser verdünnt und auf ein Pathogen und/oder den Ort seines Auftretens und den Ort, an dem eine Pflanzenkrankheit auftritt, aufgesprüht.

(b) Emulgierbares Konzentrat

30 Teile der Verbindung Nr. 2 der Erfindung, 55 Teile Xylol, 8 Teile Polyoxyethylen-alkylphenylether und 7 Teile Calciumalkylbenzolsulfonat werden unter Rühren miteinander vermischt, wodurch ein emulgierbares Konzentrat hergestellt wird. Dieses wird mit Wasser verdünnt und auf ein Pathogen und/oder den Ort seines Auftretens und den Ort, an dem eine Pflanzenkrankheit auftritt, aufgesprüht.

(c) Stäubemittel

2 Teile der Verbindung Nr. 3 der Erfindung und 98 Teile Tonpulver werden pulverisiert und gemischt, wodurch ein Stäubemittel hergestellt wird. Dieses wird über einem Pathogen und/oder dem Ort seines Auftretens und dem Ort, an dem eine Pflanzenkrankheit auftritt, ausgestreut.

(d) Stäubemittel

Die Verbindung Nr. 4 der Erfindung (1,5 Teile), 0,5 Teile Isopropylhydrogenphosphat und 98 Teile Tonpulver werden pulverisiert und gemischt, wodurch ein Stäubemittel hergestellt wird. Dieses wird über einem Pathogen und/oder dem Ort seines Auftretens und dem Ort, an dem eine Pflanzenkrankheit auftritt, ausgestreut.

(e) Granulat

Wasser (25 Teile) wird zu einer Mischung aus 10 Teilen der Verbindung Nr. 3 der Erfindung, 30 Teilen Bentonit

(Montmorillonit), 58 Teilen Talkum und 2 Teilen Lignosulfonat zugesetzt, und das Gemisch wird gut geknetet. Die Mischung wird mittels eines Extruder-Granulators zu einem Granulat mit einer Korngröße von 0,43 bis 2,0 mm (10 bis 40 mesh) verarbeitet, das dann bei 40°C bis 50°C getrocknet wird, wodurch ein Granulat gebildet wird. Das Granulat wird über einem Pathogen und/oder dem Ort seines Auftretens und dem Ort, an dem eine Pflanzenkrankheit auftritt, ausgestreut.

0173918

## P a t e n t a n s p r ü c h e

1. Substituierte Phenylsulfonylazole der Formel (I)

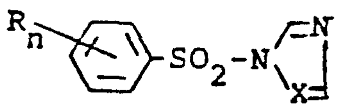

(I)

,

in der

X N oder eine CH-Gruppe bezeichnet,

R Halogen, Niederalkyl, Niederalkoxy, Trifluoromethyl, Nitro, gegebenenfalls mit Halogen substituiertes Phenyl oder Cycloalkyl bezeichnet und

n 2 oder 3 bezeichnet und weiterhin in dem Fall, in dem R nicht eine Methyl-Gruppe ist, auch 1 bezeichnet.

2. Substituierte Phenylsulfonylazole der Formel (I) nach Anspruch 1, in der

X eine CH-Gruppe oder Stickstoff bezeichnet,

R Chlor, Brom, Alkyl mit 1 bis 4 Kohlenstoff-Atomen, Alkoxy mit mit 1 bis 4 Kohlenstoff-Atomen, Nitro, Phenyl und/oder Cycloalkyl mit 5 bis 6 Kohlenstoff-Atomen bezeichnet und

n 2 oder 3 bezeichnet und weiterhin in dem Fall, in dem R nicht eine Methyl-Gruppe ist, auch 1 bezeichnet.

3. Substituierte Phenylsulfonylazole der Formel (I) nach Anspruch 1, in der
X eine CH-Gruppe bezeichnet,
R Chlor, Brom, Methyl, Ethyl, Nitro oder Phenyl bezeichnet und
n 2 bezeichnet und weiterhin in dem Fall, in dem R nicht eine Methyl-Gruppe ist, auch 1 bezeichnet.

4. Verfahren zur Herstellung substituierter Phenylsulfonylazole der Formel (I)

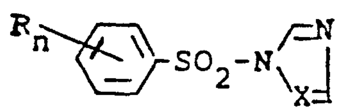

(I)

,

in der
X N oder eine CH-Gruppe bezeichnet,
R Halogen, Niederalkyl, Niederalkoxy, Trifluoromethyl, Nitro, gegebenenfalls mit Halogen substituiertes Phenyl oder Cycloalkyl bezeichnet und
n 2 oder 3 bezeichnet und weiterhin in dem Fall, in dem R nicht eine Methyl-Gruppe ist, auch 1 bezeichnet,

dadurch gekennzeichnet, daß ein substituiertes Benzolsulfonylhalogenid der Formel (II)

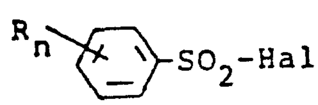

(II)

,

in der R und n die im Vorstehenden angegebenen Bedeutungen haben und Hal ein Halogen-Atom bezeichnet, mit einem Azol der allgemeinen Formel (III)

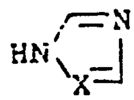

,

in der X die angegebene Bedeutung hat, umgesetzt wird, gegebenenfalls in Gegenwart von Säure-Acceptoren und inerten Lösungsmitteln.

5. Fungizide Mittel, enthaltend wenigstens ein substituiertes Phenylsulfonylazol der allgemeinen Formel (I) nach Anspruch 1 bis 4.

6. Pflanzenschutzmittel, enthaltend wenigstens ein substituiertes Phenylsulfonylazol der allgemeinen Formel (I) nach Anspruch 1 bis 4.

7. Verwendung substituierter Phenylsulfonylazole der Formel (I) nach Anspruch 1 bis 4 zur Bekämpfung von Pflanzenkrankheiten.

8. Verfahren zur Bekämpfung von Pflanzenkrankheiten, dadurch gekennzeichnet, daß ein substituiertes Phenylsulfonylazol der Formel (I) nach Anspruch 1 bis 4, entweder allein oder im Gemisch mit einem Verdünnungsmittel und/oder einem oberflächenaktiven Mittel und, sofern weiter erforderlich, einem Stabilisator, einem Haftmittel oder einem synergistischen Mittel zur Anwendung gebracht wird.

9. Verfahren zur Herstellung fungizider Mittel für Landwirtschaft und Gartenbau, dadurch gekennzeichnet, daß substituierte Phenylsulfonylazole der Formel (I) nach Anspruch 1 bis 4 bis Streckmitteln und/oder oberflächenaktiven Mitteln vermischt werden.